# FASCICULE DE BREVET EUROPEEN

(11) **EP 2 590 700 B1**
(45) Date de publication et mention de la délivrance du brevet: **14.10.2015**
(21) Numéro de dépôt: 11803200.2
(22) Date de dépôt: 26.05.2011
(51) Int. Cl.: A61M 15/00

(54) **INHALATEUR UNIDOSE DE POUDRE SÈCHE POUR BLISTER PELABLE**
EINZELDOSIS-TROCKENPULVERINHALATOR FÜR EINE ABLÖSBARE BLISTERPACKUNG
SINGLE-DOSE DRY POWDER INHALER FOR A PEELABLE BLISTER PACK

(30) Priorité: 09.07.2010 FR 1055623
(43) Date de publication de la demande: 15.05.2013
(73) Titulaire: Aptar France SAS, 27110 Le Neubourg (FR)
(72) Inventeur: PARDONGE, Jean-Marc, 76520 Les Authieux Sur Port Saint Ouen (FR)
(74) Mandataire: CAPRI
(86) Numéro de dépôt international: PCT/FR2011/051201
(87) Numéro de publication internationale: WO 2012/004485

(56) Documents cités:
- WO-A1-03/082389
- WO-A1-2007/129127
- WO-A1-2007/129128

## Description

La présente invention concerne un inhalateur unidose de poudre sèche.

Les inhalateurs de poudre, notamment du type pharmaceutique, sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Un autre type d'inhalateur consiste à disposer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Concernant l'ouverture des réservoirs individuels, il a été proposé de peler, décoller ou percer la couche de fermeture. Un autre problème qui se pose avec les inhalateurs pourvus de bande de blisters est lié au déplacement de la bande, et au stockage de la partie utilisée de la bande. Ainsi, selon la longueur de la bande et/ou l'épaisseur des blisters, un espace important peut s'avérer nécessaire et tout blocage de la bande de blisters peut empêcher le bon fonctionnement de l'inhalateur. Par ailleurs, lorsque le dispositif d'avancée de la bande tire en même temps sur l'extrémité avant de la bande pour éviter un mauvais enroulement, il peut se poser un problème au fur et à mesure des actionnements en raison notamment du diamètre de la bande usée enroulée qui augmente progressivement. Les inhalateurs multidoses et les inhalateur contenant une bande de blisters sont donc généralement des dispositifs complexes, constituées d'un grand nombre de pièces, et donc coûteux à fabriquer et à assembler. Pour réaliser des dispositifs moins complexes et donc moins coûteux, il a été proposé des inhalateurs unidoses comportant un seul réservoir individuel, tel qu'une capsule, qui est à charger dans l'inhalateur avant chaque utilisation de celui-ci. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. De plus, d'autres inconvénients spécifiques à ces inhalateurs à capsules sont apparus. Ainsi, les performances fluidiques sont très différentes par rapport aux inhalateurs multidoses utilisant par exemple des bandes de blisters pelables, tels que décrits notamment dans les documents US5873360 et US5590645. Or, ces inhalateurs multidoses à bande de blisters pelable sont largement utilisés, notamment par un inhalateur appelé « Diskus » commercialisé par la société Glaxo. Il peut donc être souhaitable, notamment pour les médicaments génériques, de proposer un inhalateur simplifié et peu coûteux, mais qui, au moment de l'inhalation par l'utilisateur, reproduit le plus possible les caractéristiques fluidiques des inhalateurs multidoses à bande de blisters pelable. Les documents WO 2007/129127 et WO 2007/129128 décrivent des dispositifs de l'art antérieur, qui présentent notamment l'inconvénient d'un assemblage peu aisé d'un blister dans un inhalateur unidose, ce qui rend son utilisation compliqué par exemple en cas de crise ou pour des personnes handicapées.

La présente invention a pour but de fournir un inhalateur unidose de poudre sèche, qui ne reproduit pas les inconvénients susmentionnés.

En particulier, la présente invention a pour but de fournir un tel inhalateur qui soit simple et peu coûteux à fabriquer et à assembler, et fiable d'utilisation.

La présente invention a donc pour objet un inhalateur unidose de poudre sèche tel que décrit dans la revendication 1. Des variantes avantageuses sont décrites dans les revendications dépendantes.

Ces caractéristiques et avantages et d'autres de la présente invention apparaîtront plus clairement au cours de la description détaillée suivante, faite en référence aux dessins joints, donnés à titre d'exemples non limitatifs, sur lesquels
- la figure 1 est une vue schématique éclatée en perspective d'un dispositif de distribution selon un premier mode de réalisation avantageux,
- la figure 2 est une vue schématique en perspective du dispositif de la figure 1, en position fermée,
- la figure 3 est une vue schématique en section transversale du dispositif de la figure 2,
- la figure 4 est une vue similaire à celle de la figure 2, en position ouverte,
- la figure 5 est une vue schématique en section transversale du dispositif de la figure 4,
- la figures 6 est une vue similaire à celle de la figure 5, en cours de mise en place d'un blister dans l'inhalateur,
- la figures 7 est une vue similaire à celle de la figure 6, après mise en place d'un blister dans l'inhalateur,
- la figure 8 est une vue schématique en section transversale d'un blister utilisable avec la présente invention,
- la figure 9 est une vue schématique en section transversale du dispositif de la figure 7,
- la figures 10 est une vue similaire à celle de la figure 9, après fermeture de l'inhalateur, avec un blister fermé disposé dans l'inhalateur,
- la figure 11 est une vue de détail de la figure 10
- la figure 12 est une vue similaire à celle de la figure 7, en position fermée, en cours d'ouverture du blister,
- la figure 13 est une vue similaire à celle de la figure 10, avec le blister ouvert,
- la figure 14 est une vue schématique en section transversale d'une partie du dispositif de la figure 13, en cours d'inhalation,
- la figure 15 est une vue schématique éclatée en perspective d'un dispositif de distribution selon un second mode de réalisation avantageux,
- la figure 16 est une vue schématique d'un blister utilisable avec ce second mode de réalisation avantageux,
- la figure 17 est une vue schématique en section transversale du dispositif de la figure 15, en position ouverte, après insertion du blister de la figure 16,
- la figure 18 est une vue similaire à celle de la figure 17, après fermeture de l'inhalateur, avec un blister fermé disposé dans l'inhalateur, et
- la figure 19 est une vue de détail de la figure 18,

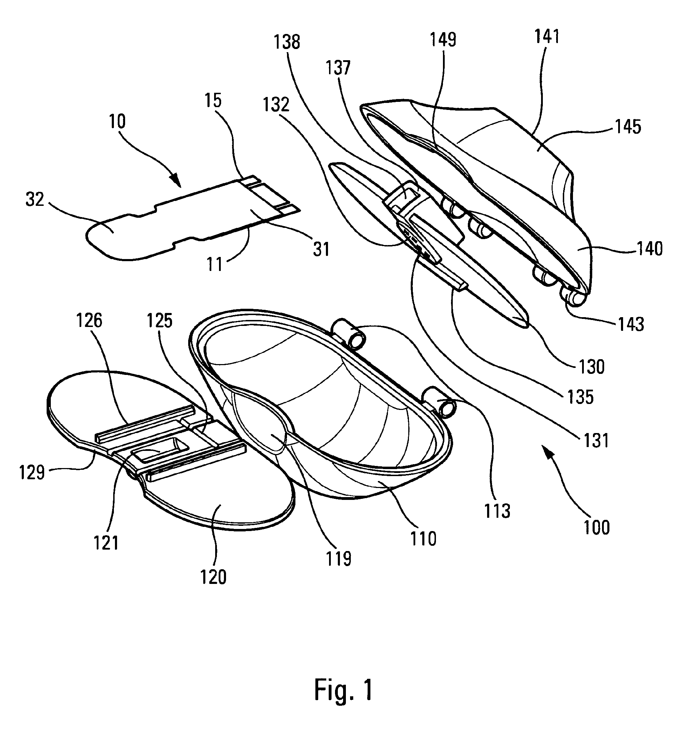

Les figures 1 à 14 décrivent un premier mode de réalisation avantageux de l'invention. Dans ce mode de réalisation, l'inhalateur 100 comporte un corps 110 et un capot pivotant 140 assemblé de manière pivotante sur le corps 110, par exemple via des axes de pivotement 113, 143. Le capot 140 comporte un orifice de distribution 141, formé de préférence au niveau d'un embout buccal 145 autour duquel l'utilisateur placera sa bouche pour inhaler.

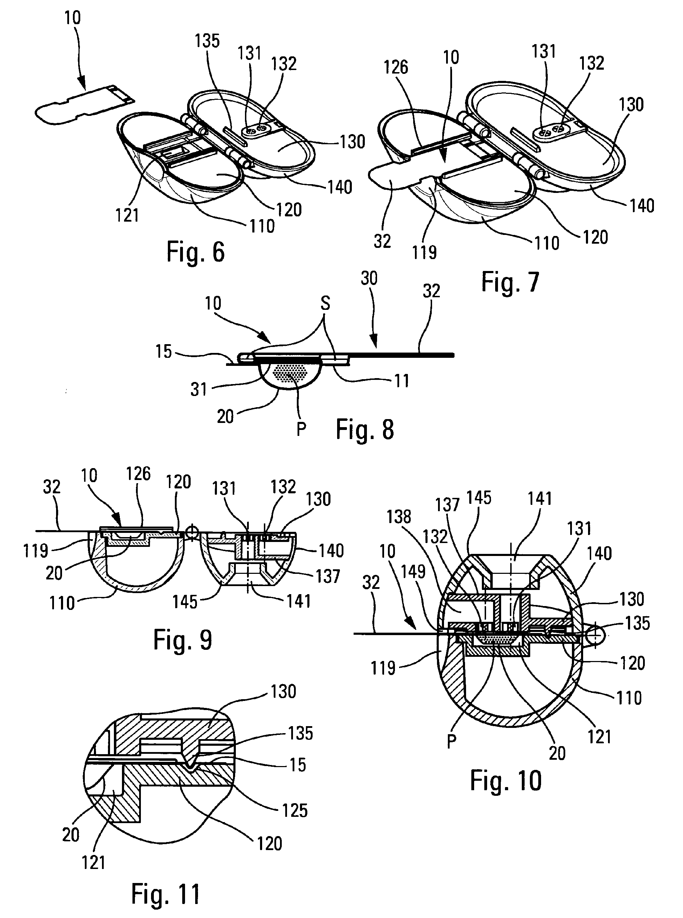

L'inhalateur unidose 100 est destiné à recevoir un blister pelable 10. Un tel blister plein est à charger dans l'inhalateur avant chaque utilisation, et après chaque inhalation, le blister vide est évacué hors dudit inhalateur. Un exemple d'un tel blister 10 est représenté sur la figure 8. Ce blister comporte une cavité 20 contenant une dose de poudre P, et une couche de fermeture 30 pourvue d'une partie avant de préhension 32 par laquelle l'utilisateur peut ouvrir le blister, comme cela sera décrit plus en détails ci-après.

Le corps 110 comporte une plaque porte-blister 120 fixée audit corps. Cette plaque porte-blister 120 comporte des moyens de réception d'un blister. En particulier, ladite plaque porte-blister 120 comporte une cavité 121 destinée à recevoir la cavité 20 d'un blister 10. Avantageusement, ladite plaque porte-blister 120 comporte également des moyens de positionnement 126, tels que des rails, pour positionner correctement ledit blister 10.

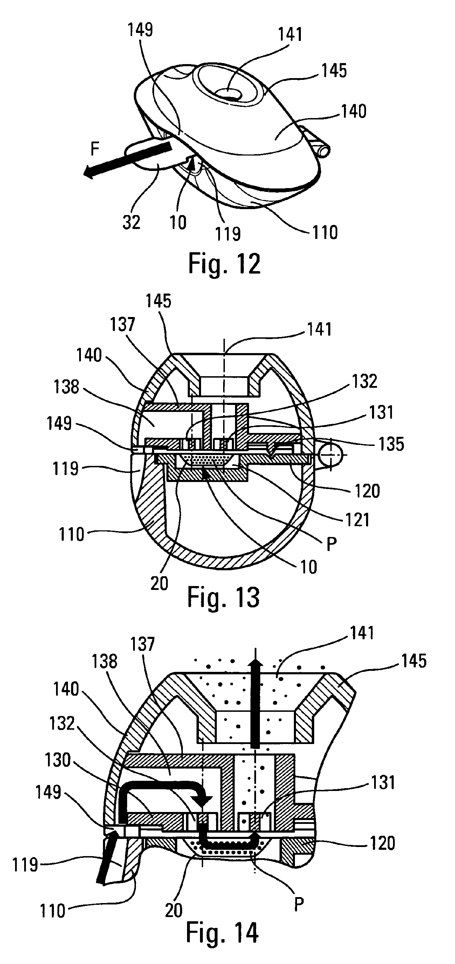

Le capot 140 comporte une plaque porte-moteur 130 fixée audit capot. Cette plaque porte-moteur 130 comporte un moteur formé d'un passage d'air 138 menant à une première ouverture 132. Le passage 138 est défini par une projection 137 creuse reliée d'un coté à l'extérieur et de l'autre coté à ladite première ouverture 132. A coté de cette première ouverture 132 se trouve une seconde ouverture 131 disposée face à l'orifice de distribution en position fermée de l'inhalateur. Ces deux ouvertures 132, 131 sont disposées, en position fermée, face à la cavité 121 de la plaque porte-blister 120, et donc face à une cavité 20 d'un blister 10 lorsque celui-ci est disposé dans l'inhalateur. Lorsque l'utilisateur inhale, il va donc créer un flux d'air qui va passer dans le passage 138, puis pénétrer dans le blister 10 par ladite première ouverture 132, traverser la cavité 20 dudit blister, et ressortir chargé de la poudre à travers la seconde ouverture 131, pour être inhalé à travers l'orifice de distribution 141. Ceci est illustré sur la figure 14, le cheminement du flux d'air étant représenté par les flèches. La plaque porte-moteur 130 comporte avantageusement une projection de blocage 135 adaptée à coopérer avec le blister 10 et la plaque porte-blister 120, pour bloquer ledit blister au moment où l'utilisateur va tirer sur la partie de préhension 32 pour l'ouvrir. Avantageusement, cette projection de blocage 135 va coincer une partie arrière 15 du blister 10, qui n'est pas recouverte par la partie de fermeture, dans une rainure 125 de forme correspondante prévue dans la plaque porte-blister 120, lorsque le capot 140 est refermé. Cette position de blocage est notamment visible sur la figure 11.

Le corps 110 comporte avantageusement un renfoncement frontal 119, et la plaque porte-blister 120 comporte un renfoncement correspondant 129. Le capot 140 comporte avantageusement une zone frontale 149 adaptée à coopérer avec ledit renfoncement frontal 129 du corps 110 pour d'une part faciliter l'ouverture dudit capot 140, et d'autre part favoriser le cheminement du flux d'inhalation lors de l'inhalation de l'utilisateur, en reliant l'extérieur de l'inhalateur audit passage 138.

Ainsi, comme visible notamment sur la figure 1, le dispositif selon ce mode de réalisation est constitué de quatre parties principales, à savoir le corps 110, la plaque porte-blister 120, le capot 140 et la plaque porte-moteur 130.

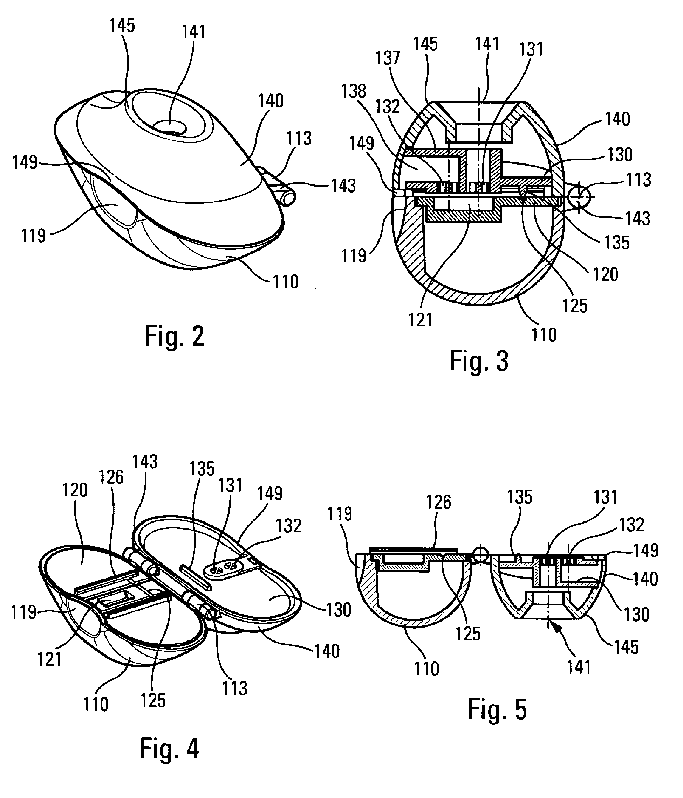

Les figures 2 à 14 illustrent un cycle de fonctionnement du dispositif selon ce mode de réalisation.

Les figures 2 et 3 montrent le dispositif en position fermée sans blister disposé dans l'inhalateur. L'utilisateur ouvre alors le capot 140, comme illustré par les figures 4 et 5, et dispose simplement un blister 10 sur la plaque porte-blister 120, comme illustré par les figures 6 et 7. Le blister n'a pas besoin d'être assemblé autour d'un profil de blocage, comme cela est décrit notamment dans les documents WO 2007/129127 et WO 2007/129128. Le capot 140 est alors refermé comme représenté sur les figures 9 et 10, ce qui bloque le blister 10 dans l'inhalateur, comme expliqué précédemment. Dans cette position fermée avec un blister dans l'inhalateur, la partie de préhension 32 du blister 10 fait saillie hors de l'inhalateur, comme visible sur la figure 10. L'utilisateur peut alors tirer sur ladite languette dans le sens de la flèche F sur la figure 12, ce qui provoque le pelage de ladite couche de fermeture 30 de ladite couche de cavité 11 du blister 10, et expose donc l'intérieur de la cavité 20. La figure 13 représente l'inhalateur fermé avec un blister ouvert disposé à l'intérieur. L'utilisateur n'a alors plus qu'à inhaler la poudre.

Avantageusement, le blister 10 comporte une couche de cavité 11 définissant la cavité 20 recevant la poudre P, et une couche de fermeture 30 qui comporte deux parties. Une première partie, formant partie de fermeture 31, qui est scellée S sur la couche de cavité 11. Avantageusement, ce scellage est réalisé de manière thermique pour assurer une étanchéité parfaite. Bien entendu, d'autres types de scellage peuvent être envisagés. La couche de fermeture 30 comporte également une seconde partie, formant partie de préhension 32. Cette partie de préhension 32, qui est de préférence réalisée d'une pièce monobloc avec la partie de fermeture 31, est repliée au-dessus de ladite partie de fermeture 31, et donc également au-dessus de ladite couche de cavité 11. Ses dimensions sont prévues de telle sorte qu'elle s'étend au-delà de ladite couche de cavité 11, pour former ainsi une languette que l'utilisateur pourra saisir afin d'ouvrir le blister 10. Si l'utilisateur tire sur la partie de préhension 32 dans le sens de la flèche F sur la figure 12, il décollera ladite couche de fermeture 30 de la couche de cavité 11 pour ouvrir la cavité 20 et ainsi rendre disponible la poudre P qu'elle contient. Avantageusement, la ligne de pliage 35 entre la partie de fermeture 31 et la partie de préhension 32, et autour de laquelle la partie de préhension 32 va être repliée au-dessus de la partie de fermeture 31, ne correspond pas exactement au bord latéral de la couche de cavité 11. Ainsi, lorsque le blister 10 est formé, comme représenté sur la figure 12, la partie arrière 15 de la couche de cavité 11 s'étend au-delà de ladite ligne de pliage 35. Cette partie arrière 15 de la couche de cavité est celle qui va être coincée par la projection de blocage 135 de la plaque porte-moteur 130 en position fermée du capot 140.

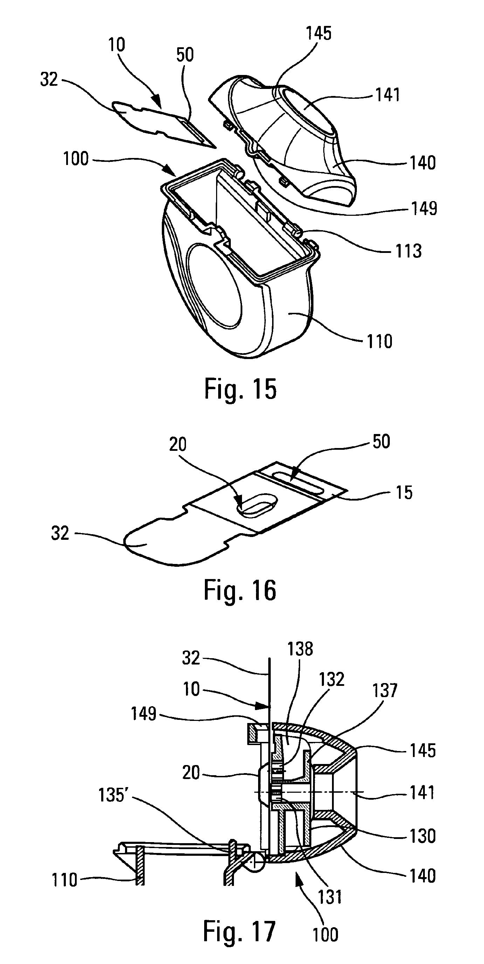

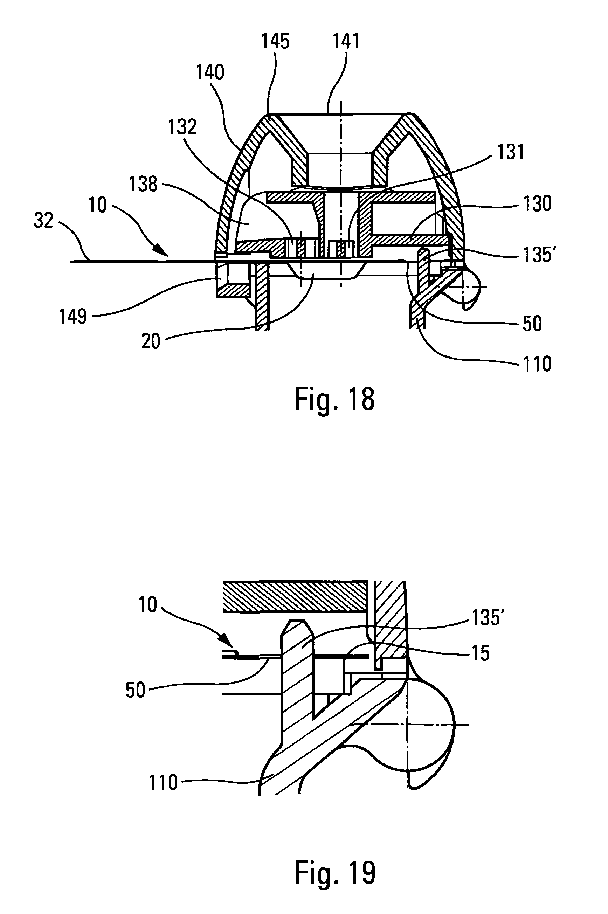

Les figures 15 à 19 illustrent un second mode de réalisation de l'invention. Le fonctionnement de ce second mode de réalisation est similaire à celui du premier mode de réalisation, mais le blister 10 est fixé sur le capot 140. Le corps 110 ne comporte plus de plaque porte-blister, et la plaque porte-moteur 130, fixée au capot 140, définit avec ledit capot 140 une ouverture d'insertion frontale 149, dont la forme peut être adaptée à celle du blister 10, comme visible sur la figure 15. Lorsque le capot est ouvert, le blister 10 peut être inséré à travers ladite ouverture 149, comme visible sur la figure 17. Ici aussi, l'insertion est simple, sans nécessiter l'emmanchement dudit blister sur un quelconque profil de blocage. Lorsque le capot 140 est refermé, une projection 135' du corps 120 vient coopérer avec une ouverture 50 prévue dans la partie arrière 15 du blister, pour bloquer ledit blister 10 dans l'inhalateur lorsque l'utilisateur procédera à l'ouverture du blister. La figure 16 illustre un exemple de blister 10 qui s'adapte à ce mode de réalisation. La figure 18 représente l'inhalateur avec le blister 10 après fermeture du capot 140, prêt à l'usage. La figure 19 illustre de manière plus détaillée et agrandie la projection 135' qui traverse l'ouverture 50 du blister 10. Les autres caractéristiques du dispositif et le mode d'utilisation sont similaires à ceux décrits en référence au premier mode de réalisation. Ce second mode de réalisation est avantageux car plus simple, étant notamment réalisé avec moins de pièces constitutives.

Avantageusement, la forme de l'embout buccal et le moteur sont réalisés de manière similaire à l'inhalateur « Diskus » de la société Glaxo, pour ainsi reproduire des performances fluidiques et des propriétés d'inhalation de cet inhalateur lorsque l'utilisateur va inhaler. Le fait d'utiliser un blister pelable, dont la forme de la cavité et la contenance sont similaires sinon identiques aux blisters utilisés sur les bandes de blisters pelables du « Diskus », permet de reproduire les mêmes performances au moment de l'inhalation, avec toutefois un inhalateur beaucoup plus simple de conception, puisque ne comportant ni moyens de déplacement de la bande de blister, ni moyens d'ouverture desdits blisters.

Diverses modifications sont également possibles pour un homme du métier sans sortir du cadre de la présente invention tel que défini par les revendications annexées. En particulier, les diverses caractéristiques et fonctionnalités du dispositif décrites en référence aux dessins peuvent être combinées entre elles d'une quelconque façon appropriée.

## Revendications

1. Inhalateur unidose de poudre sèche (100), comportant un corps (110), un capot (140) pivotant sur ledit corps, ledit capot comportant un orifice de distribution (141) à travers lequel l'utilisateur inhale, ledit inhalateur (100) recevant un blister (10) comportant une cavité (20) contenant une dose de poudre (P) et une couche de fermeture pelable (30) comportant une partie de fermeture (31), fermant de manière étanche ladite cavité (20), et une partie de préhension (32) s'étendant hors dudit inhalateur pour permettre l'ouverture dudit blister par pelage de ladite couche de fermeture par traction directe de l'utilisateur sur ladite partie de préhension (32), **caractérisé en ce que** ledit capot (140) est adapté à recevoir ledit blister (10) en position d'ouverture dudit capot (140), ledit corps (110) comportant une projection de blocage (135') adaptée à coopérer avec une partie arrière (15) dudit blister (10) en position fermée dudit capot (140), pour bloquer ledit blister (10) dans l'inhalateur lorsque l'utilisateur ouvre ledit blister (10) en tirant sur la partie de préhension (32) pour peler ladite couche de fermeture (30).

2. Inhalateur selon la revendication 1, dans lequel ledit inhalateur comporte une plaque porte-moteur (130) comportant un passage (138), une première ouverture (132) et une seconde ouverture (131), de sorte que lors de l'inhalation par l'utilisateur, le flux d'inhalation va entrer dans ledit passage (138), pénétrer dans le blister ouvert par ladite première ouverture (132), et ressortir par ladite seconde ouverture (131) chargé de la poudre, en direction de l'orifice de distribution (141).

3. Inhalateur selon la revendication 2, dans lequel ledit blister (10) est assemblé sur ladite plaque porte-moteur (130) en position ouverte dudit capot (140).

4. Inhalateur selon la revendication 3, dans lequel ladite plaque porte-moteur (130) définit avec ledit capot (140) une ouverture (149) pour l'insertion du blister (10).

5. Inhalateur selon la revendication 3 ou 4, dans lequel ladite projection (135') coopère avec une ouverture (50) réalisée dans la partie arrière (15) du blister (10) pour bloquer ledit blister (10) dans l'inhalateur en position fermée dudit capot (140).

## Patentansprüche

1. Einzeldosis-Trockenpulverinhalator (100), umfassend einen Körper (110), eine Kappe (140), die auf dem Körper schwenkbar ist, wobei die Kappe eine Ausgabeöffnung (141) umfasst, durch welche der Benutzer inhaliert, wobei der Inhalator (100) einen Blister (10) aufnimmt, der einen Hohlraum (20), welcher eine Dosis Pulver (P) enthält, und eine abziehbare Verschlussschicht (30) umfasst, die einen Verschlussteil (31), der den Hohlraum (20) dichtend verschließt, und einen Greifteil (32) umfasst, der sich von dem Inhalator nach außen erstreckt, um das Öffnen des Blisters durch Abziehen der Verschlussschicht durch direktes Ziehen an dem Greifteil (32) durch den Benutzer zu ermöglichen, **dadurch gekennzeichnet, dass** die Kappe (140) dafür ausgelegt ist, in der Öffnungsposition der Kappe (140) den Blister (10) aufzunehmen, wobei der Körper (110) einen Sperrvorsprung (135') umfasst, der dafür ausgelegt ist, in der geschlossenen Position der Kappe (140) mit einem hinteren Teil (15) des Blisters (10) zusammenzuwirken, um den Blister (10) in dem Inhalator zu sperren, wenn der Benutzer den Blister (10) öffnet, indem er an dem Greifteil (32) zieht, um die Verschlussschicht (30) abzuziehen.

2. Inhalator nach Anspruch 1, wobei der Inhalator eine Antriebsträgerplatte (130) umfasst, die einen Kanal (138), eine erste Öffnung (132) und eine zweite Öffnung (131) umfasst, so dass bei Inhalation durch den Benutzer der Inhalationsstrom in den Kanal (138) eintritt, in den durch die erste Öffnung (132) geöffneten Blister eindringt und mit Pulver beladen wieder durch die zweite Öffnung (131) in Richtung zur Ausgabeöffnung (141) austritt.

3. Inhalator nach Anspruch 2, wobei der Blister (10) in der geöffneten Position der Kappe (140) auf der Antriebsträgerplatte (130) montiert ist.

4. Inhalator nach Anspruch 3, wobei die Antriebsträgerplatte (130) mit der Kappe (140) eine Öffnung (149) zum Einführen des Blisters (10) definiert.

5. Inhalator nach Anspruch 3 oder 4, wobei der Vorsprung (135') mit einer Öffnung (50) zusammenwirkt, die in dem hinteren Teil (15) des Blisters (10) verwirklicht ist, um den Blister (10) in dem Inhalator in der geschlossenen Position der Kappe (140) zu sperren.

## Claims

1. A single-dose dry powder inhaler (100), comprising a body (110), a cover (140) pivoting on said body, said cover comprising a dispensing orifice (141) through which the user inhales, said inhaler (100) receiving a blister pack (10) comprising a recess (20) containing a dose of powder (P) and a peelable closing layer (30) comprising a closing portion (31), which sealingly closes said recess (20), and a gripping portion (32) extending out of said inhaler to enable said blister pack to be opened by peeling off said closing layer by the user directly pulling on said gripping portion (32), **characterized in that** said cover (140) is adapted to receive said blister pack (10) in the open position of said cover (140), said body (110) comprising a locking projection (135') adapted to cooperate with a rear portion (15) of said blister pack (10) in the closed position of said cover (140) so as to block said blister pack (10) in the inhaler when the user opens said blister pack (10) by pulling on the gripping portion (32) to peel off said closing layer (30).

2. The inhaler according to claim 1, wherein said inhaler comprises a motor holder plate (130) comprising a passage (138), a first opening (132) and a second opening (131), such that during inhalation by the user, the inhalation flow will enter into said passage (138), penetrate the open blister pack via said first opening (132), and exit via said second opening (131) loaded with powder, in the direction of the dispensing orifice (141).

3. The inhaler according to claim 2, wherein said blister pack (10) is assembled on said motor holder plate (130) in the open position of said cover (140).

4. The inhaler according to claim 3, wherein said motor holder plate (130) defines with said cover (140) an opening (149) for inserting the blister pack (10).

5. The inhaler according to claim 3 or 4, wherein said projection (135') cooperates with an opening (50) made in the rear part (15) of the blister pack (10) to block said blister pack (10) in the inhaler in the closed position of said cover (140).
